# EUROPEAN PATENT APPLICATION

(11) **EP 0 539 610 A1**
(43) Date of publication of application: **05.05.1993**
(21) Application number: 91118269.9
(22) Date of filing: 26.10.1991
(51) Int. Cl.: G01N 33/50, G01N 33/68

(54) **Method and composition for determining the immunological activity of solutions containing active substances**

(71) Applicant: Torf Establishment, FL-9490 Vaduz (LI)
(72) Inventor: Inglot, Anna, Wroclaw (PL); Blach-Olszewska, Zofia, Wroclaw (PL)
(74) Representative: Büchel, Kurt F., Dr.

(57) **Abstract**

The immunological activity of certain bioactive substances is determined by treating a human peripheral blood leukocyte (PBL) culture or a Bulb/C mice resident peritoneal cells (RPC), such as abdominal membrane macrophage suspension, with a solution of the substance to be tested in order to induce production of cytokines which then are determined according to standard identification methods amplification of the results is achieved by admixing - to the solution to be tested - a non-steroidal anti-inflammatory drug, preferably a substance selected from the group of selenoorganic compounds such as Ebselen (R) of Nattermann (Rhône-Poulenc), indo-methacin and the p-chlorophenyl-amide of 3-methyl-5-benzoyl-amino-isothiazole-4-carboxylic acid.

## Description

The present invention relates to a method and composition as defined in the introductory part of claim 1.

The method allows to determine whether a tested substance is able to induce production of different cytokines and permits a quick and efficient check of the properties of certain substances, namely their immunological activity, at each stage of production, separation, purification and formulation into final compositions. The present invention provides also a composition suitable for pharmaceutical applications in humans and animals.

Tests used until now to determine immunological activity are numerous but difficult to carry out. When a new therapeutical composition having an immunological effect is introduced and clinically tested, its immunological effectiveness is evaluated by monitoring all numerous, well known and analytically determinable features of immunological systems employing standard methods. Once the activity was proven, there emerges the necessity for representative single tests enabling a quick check of the status of a patient in terms of his or her immunological response.

On the other hand, technological processes for manufacturing, purification or separation of immunologically active substances such as in particular extracts from raw peat are usually multi-step ones, and there is always the need for establishing a reliable and fast test enabling a thorough control of the production. Moreover, since the substances are frequently of a complex nature as it is the case with peat extracts, it is important to have a similar test for determining the activity of individual fractions of the same. It is also necessary to find out a microscale testing method, since the products to be analysed are very costly.

The following three basic findings allowed to solve the above described problems.
1. Some immunoactive peat extracts induce the production of cytokines such as interferons and tumor necrosis factors in a peripheral blood leukocyte in in-vitro cultures and also that the induction is dependant on the dose.
2. Interferon β is produced by mice abdominal membrane macrophages when treated with immunoactive peat extract; also this process is dependant on the dose. Such a reaction is observed when immunoactive peat extract, its fractions or intermediate solutions obtained in a process of separation of the extract from raw peat are tested.
3. Certain peat extracts, when tested in-vitro as mentioned above show a synergistic effect in compositions with known immunomodulators such as selenium-organo-compounds, the p-chlorophenylamide of 3-methyl-5-benzoylamino-isothiazol-4-carboxylic acid (Vratizolin^{(R)} of Polfa, Poland) and indomethacin. This allows the use of much smaller quantities of immunologically active substances to show their activity.

The invention lies in the steps described in the characterizing clause of claim 1. According to the invention, a peripheral blood leukocyte culture is a short term culture prepared from fresh leukocytes of healthy humans with a nutrient medium suitable for tissue culture. The preferred density of a ready-for-use PBL culture is approx. 8 x 10⁶ leukocytes/ml. The solution to be tested is preferably used in a concentration of 0,1 - 200 µg/ml. The method is in particular suitable for testing peat extracts.

A micro-scale determination is possible when the solution of peat extract to be tested is admixed with a known immunomodulating agent such as selenium-organo-compounds, Vratizolin^{(R)} or indomethacin.

In the instant invention, different sera, which recognise individual induced cytokines, are employed. These sera are known and used for testing and standardisation of different cytokines. Identification of the induced cytokines should take place at the moment of completion of the cytokine formation and prior to its proteolysis. Among different cytokines induced, some are formed slowly and remain stable in the solution (as for example interferons) while others are formed rapidly and are susceptible to proteolysis as for example tumor necrosis factors.

The method as described may be employed also for the determination of an immunological response of a human individual to a certain immunoactive substance, provided that immunological response is not stimulated by other factors as for example viral or bacteria infection.

The present invention also provides for a testing method which is suitable for quick monitoring of technological processes for the production, purification, separation or the like of the immmunoactive substances. For instance is a solution of Bulb/C mice abdominal membrane macrophages treated with the solution to be tested, and thus induces mice interferon β as well as tumor necrosis factor. Both may be detected and qualitatively determined according to standard identification methods. In the method, a solution of fresh mice abdominal membrane macrophage cells, containing approx. 1 x 10⁶ cells/ml is used. The solution to be tested is prepared in a nutrient medium Eagle,RPMI-1640 with the addition of 10% of fetal calf serum. The results obtained are evaluated against a calibration curve prepared with a model substance. As a model substance, a sample of the same immunologically active substance tested in a traditional way may be used. Again, the method is particularly suitable for monitoring the process for obtaining peat extracts as well as analysing its fractions. Similarly, as in the case of the previously described method, a micro-scale determination is possible when a non-steroidal anti-inflammatory drug, preferably a substance selected from the group of seleno-organic compounds such as Ebselen^{(R)} of Nattermann (Rhône-Poulenc), indomethacin and the p-chlorophenyl-amide of 3-methyl-5-benzoyl-amino-isothiazole-4-carboxylic acid (Vratizolin^{(R)} of Nattermann, Rhône-Poulenc) or indomethacin is used. Amplification of the results is 5 - 20 times.

The method is very simple and efficient in comparison with other known methods, because it is based on a direct induction of interferon β instead - as is the case in a number of conventional tests - on a secondary effect of formulation of antibodies. Until now, it was not possible to find any similar method due to the fact that the immunosystem of mice significantly differs from the human immunosystem and tests recognised as indicative for human immunologic response - such as the presence of interferons in the tissues of lien or lymphoglands etc. - do not work with the best laboratory test animals, i.e. with mice of Bulb/C type.

It was now found out that a clear immunological response of the Bulb/C mice can be found when abdominal membrane macrophages are chosen as a biological material for testing. Fresh solution of the mice abdominal membrane macrophages is used. For each test it is sufficient to sacrifice just three animals. Results are obtained within several hours. The biological material may also be cultured and a corresponding line of macrophages be established.

The method according to the present invention employ the use of biological material, namely a peripheral blood leukocyte culture or Bulb/C mice abdominal membrane macrophage solution, according to the information given above. Presence and concentration of each cytokine is determined by methods for testing and standardisation of interferons known from "Methods in Enzymologxy", 1986, vol.119, part C: "Interferon", edited by Sidney Pestka. Determination of the whole spectrum of cytokines induced identifies the immunological status of a patient in a complex way.

For the proper evalution of the results, it is essential that the biological material for carrying out both methods according to the present invention is selected from the proper donors. In case of human leukocytes, samples taken from individuals (rare case) who show a hypo-reactivity, i.e. a low level of immunological response, should be excluded from the evaluation. In case of the Bulb/C mice abdominal membrane macrophages solution, samples taken from the additionally stimulated animals (for example infected ones) should also be excluded from the evaluation.

To show effectiveness of the two tests according to the present invention, the following example is provided.

### Example:

Peat extract samples taken from different production batches numbered as shown in Tab. 1 have been tested using a human leukocytes test as described above. In the experiments, interferon (IFN) and tumor necrosis factor (TNF) have been determined qualitatively and according to the above mentioned standard procedure; their activity, as expressed in International Activity Units, was determined. Experiments were repeated a number of times quoted in Tab. 1. Result ranges and mediana are also given.

For illustration purposes, a similar test was carried out with a standardised peat preparation. At a concentration of 30 µg/ml, interferon activity was within a range of 30 - 300 International Activity Units, while at a concentration of 100 µg/ml, it was 300 - 1000 International Activity Units.

Intermediate concentrations showed a linear relation between dose and response.

## Claims

1. Method for determining the immunological activity of certain bioactive substances, characterised in that a human peripheral blood leukocyte (PBL) culture or a Bulb/C mice resident peritoneal cells (RPC), such as abdominal membrane macrophage suspension is treated with a solution of the substance to be tested in order to induce production of cytokines which then are determined according to standard identification methods.

2. Method according to claim 1, characterised in that a human PBL culture is used and induces the production of different cytokines whereafter each of the induced cytokines is identified qualitatively and quantitatively using a set of different - preferably known - sera which recognize individual cytokines.

3. Method according to claim 1 or 2, characterised in that the human PBL culture is prepared with a nutrient medium suitable for tissue culture and that the density of the ready-for-use PBL culture is approx. 8 x 10⁶ leukocytes/ml.

4. Method according to any of claims 1 to 3, characterised in that the solution to be tested is used in a concentration of 0,1 - 200 µg/ml.

5. Method according to any of claims 1 to 4, characterised in that each cytokine is identified quickly enough to avoid proteolysis of the cytokine.

6. Method according to claim 1, characterised in that a PBL or RPC cytokines, suspension is used, and the bioactive substances induce the production of cytokines' mainly interferons and tumor necrosis factors.

7. Method according to claim 6, characterised in that the RPC suspension contains approx. 1 - 2 x 10⁶ cells/ml.

8. Method according to claim 6 or 7, characterised in that the solution to be tested is prepared in a nutrient medium Eagle,RPMI-1640 with an addition of 10 % of fetal calf serum.

9. Method according to any of the preceding claims, characterised in that the results obtained are evaluated against a calibration curve.

10. Method according to any of the preceding claims, characterised in that peat extracts are tested.

11. Method according to any of the preceding claims, characterised in that amplification of the results is achieved by admixing - to the solution to be tested - a non-steroidal anti-inflammatory drug, preferably a substance selected from the group of seleno-organic compounds such as Ebselen (R) of Nattermann (Rhône-Poulenc), indomethacin and the p-chlorophenyl-amide of 3-methyl-5-benzoylamino-isothiazole-4-carboxylic acid.

12. Composition for carrying out the method according to any of claims 1 to 11, characterised in that it contains on one hand a PBL culture or a RPC suspension, and on the other hand a non-stereoidal anti-inflammatory drug, preferably a substance selected from the group of seleno-organic compounds, indo-methacin and the p-chlorophenylamide of 3-methyl-5-benzoylamino-isothiazole-4-carboxylic acid.
